# EUROPEAN PATENT APPLICATION

(11) **EP 3 690 557 A1**
(43) Date of publication of application: **05.08.2020**
(21) Application number: 20152835.3
(22) Date of filing: 21.01.2020
(51) Int. Cl.: G04C 19/00, G04G 11/00, A61B 5/00

(54) **INFORMATION PROCESSING METHOD, INFORMATION PROCESSING PROGRAM, AND INFORMATION PROCESSING SYSTEM**

(30) Priority: 28.01.2019 JP 2019011972
(71) Applicant: Panasonic Intellectual Property Management Co., Ltd., Osaka-shi, Osaka 540-6207 (JP)
(72) Inventor: MIZUNO, Etsuko, Osaka-shi, Osaka 540-6207 (JP); HARADA, Masaaki, Osaka-shi, Osaka 540-6207 (JP); KOHASHI, Yasuo, Osaka-shi, Osaka 540-6207 (JP); MICHIMORI, Akihiro, Osaka-shi, Osaka 540-6207 (JP)
(74) Representative: Grünecker Patent- und Rechtsanwälte PartG mbB

(57) **Abstract**

An information processing method causes a server to acquire a stimulus control pattern for controlling a stimulus that one or more devices give to a first user to induce the first user to fall asleep or wake up, to acquire a sleep state of a second user who is in the same space in which the first user is, to adjust the acquired stimulus control pattern, based on the sleep state of the second user, and to control the operation of the one or more devices, according to the adjusted stimulus control pattern.

## Description

### Field of the Invention

The present disclosure relates to an information processing method, an information processing program, and an information processing system that control the operation of one or more devices that control a stimulus to induce a user to fall asleep or wake up.

### Background Art

Houses each having a main bedroom furnished not with one lighting device shared by husband and wife, who sleep together in the bedroom, but with two lighting devices used respectively by the husband and wife have been increasing in number in recent years, showing a sign that such a house will become widespread in future. Under such a circumstance, an equipment control system that induces a user to have a good sleep poses a problem that when it causes two lighting devices to perform different lighting controls respectively for husband and wife, the lighting controls may disturb the husband's or wife's sleep.

To deal with such a problem, a bedroom lighting system has been proposed, according to which a night table is disposed between one bed and another bed adjacent to the one bed, the night table blocking light from a group of wake-up assist lights arranged on a head board of the other bed, (for example, see Japanese Patent No. 5260997). The night table allows the husband and wife to have their respective private spaces, and even in the case of performing different lighting controls respectively for the husband and wife, the husband's or wife's sleep is not disturbed.

Further, another lighting device has also been proposed, according to which when one person walking toward a bedroom is detected while the other person is asleep in the bedroom, a footlight in the bedroom and a footlight in a hall in front of the bedroom are turned on (for example, see Japanese Unexamined Patent Application Publication No. 2018-6087). The lighting device shows a path through which the one person walks to reach the bedroom, and prevents the other person's sleep from being disturbed when the one person enters the bedroom. According to the lighting device described in Japanese Unexamined Patent Application Publication No. 2018-6087, when the one person wakes up and the other person is still asleep, only the footlight is turned on. As a result, the other person, who is still asleep, does not have his or her sleep disturbed, and is therefore allowed to keep sleeping comfortably. Further, according to the lighting device described in Japanese Unexamined Patent Application Publication No. 2018-6087, when the one person wakes up first and then the other person wakes up later, lighting equipment is turned on in such a way as to create brightness that gives a stimulus to the other person. This gives the other person a pleasant wake-up feeling.

### Summary of the Invention

However, the above conventional techniques pose a problem that the lighting device may need additional physical equipment and a problem that an effect of facilitating falling asleep or waking up may be reduced due to control of a stimulus, such as lighting, to a specific user among a plurality of users who sleep in the same space.

The present disclosure has been conceived to solve the above problems. An object of the present disclosure is to provide an information processing method, an information processing program, and an information processing system that inhibit a reduction in an effect of facilitating falling asleep or waking up, the reduction being caused by control of a stimulus, such as lighting, to a specific user among a plurality of users who sleep in the same space, without using physical equipment.

An information processing method according to the present disclosure includes, by a computer: acquiring a stimulus control pattern for controlling a stimulus that one or more devices give to a first user to induce the first user to fall asleep or wake up; acquiring a sleep state of a second user who is in the same space in which the first user is; adjusting the acquired stimulus control pattern, based on the sleep state of the second user; and controlling an operation of the one or more devices, according to the adjusted stimulus control pattern.

According to the present disclosure, a reduction in an effect of facilitating falling asleep or waking up, the reduction being caused by control of a stimulus, such as lighting, to a specific user among a plurality of users, can be inhibited without using physical equipment even when the plurality of users sleep in the same space.

### Brief Description of the Drawings

FIG. 1 depicts an overall configuration of an equipment control system according to a first embodiment of the present disclosure;
FIG. 2 depicts a configuration of a server according to the first embodiment of the present disclosure;
FIG. 3 depicts an example of stimulus control patterns for controlling a stimulus given to a first user who wakes up first, according to the first embodiment of the present disclosure;
FIG. 4 depicts an example of a table that is stored in a reached illuminance range storage unit according to the first embodiment of the present disclosure;
FIG. 5 depicts an example of a table that is stored in the reached illuminance range storage unit when the first user and a second user are separately lighted up, according to the first embodiment of the present disclosure;
FIG. 6 depicts an example of a table that is stored in the reached illuminance range storage unit when the first user's eyes and the second user's eyes are separately lighted up, according to the first embodiment of the present disclosure;
FIG. 7 is a flowchart for explaining an operation of the server according to the first embodiment of the present disclosure;
FIG. 8 depicts another example of the stimulus control patterns for controlling the stimulus given to the first user who wakes up first, according to the first embodiment of the present disclosure;
FIG. 9 depicts a configuration of a server according to a second embodiment of the present disclosure;
FIG. 10 depicts an example of a priority level input screen that is displayed on a first portable terminal according to the second embodiment of the present disclosure, when the first user is given priority;
FIG. 11 depicts an example of a priority level input screen that is displayed on the first portable terminal according to the second embodiment of the present disclosure, when the second user is given priority;
FIG. 12 is a first flowchart for explaining an operation of the server according to the second embodiment of the present disclosure;
FIG. 13 is a second flowchart for explaining an operation of the server according to the second embodiment of the present disclosure;
FIG. 14 depicts an example of an illuminance input screen that receives input of a preferred illuminance that the first user wishes to be exposed when waking up, the illuminance input screen being displayed on the first portable terminal according to the first and second embodiments of the present disclosure; and
FIG. 15 depicts an example of stimulus control patterns for controlling a stimulus given to the first user who falls asleep later, according to a modification of the first and second embodiments.

### Description of Embodiments

### (Knowledge Underlying the Present Disclosure)

The above bedroom lighting system described in Japanese Patent No. 5260997 is provided with the night table disposed between the one bed and the other bed adjacent to the one bed, the night table blocking light from the group of wake-up assist lights arranged on the head board of the other bed.

In this manner, according to the bedroom lighting system described in Japanese Patent No. 5260997, the night table for blocking light needs to be disposed between two beds adjacent to each other, which may lead to a cost increase.

The above lighting device described in Japanese Unexamined Patent Application Publication No. 2018-6087 turns on the footlight only when the one person wakes up and the other parson is still asleep. Further, according to the lighting device described in Japanese Unexamined Patent Application Publication No. 2018-6087, when the one person wakes up first and then the other person wakes up later, lighting equipment is turned on in such a way as to create brightness that gives a stimulus to the other person.

As a result, according to the lighting device described in Japanese Unexamined Patent Application Publication No. 2018-6087, the other person who wakes up after the one person wakes up has a pleasant wake-up feeling but the one person who wakes up earlier than the other person does not have a pleasant wake-up feeling.

In order to solve the above problems, an information processing method according to one aspect of the present disclosure includes, by a computer: acquiring a stimulus control pattern for controlling a stimulus that one or more devices give to a first user to induce the first user to fall asleep or wake up; acquiring a sleep state of a second user who is in the same space in which the first user is; adjusting the acquired stimulus control pattern, based on the sleep state of the second user; and controlling an operation of the one or more devices, according to the adjusted stimulus control pattern.

In this configuration, the stimulus control pattern is adjusted based on the sleep state of the second user who is in the same space in which the first user is, and the operation of the one or more devices is controlled according to the adjusted stimulus control pattern. As a result, a reduction in an effect of facilitating falling asleep or waking up, the reduction being caused by control of a stimulus, such as lighting, to a specific user (i.e., the first user) among a plurality of users, can be inhibited without using physical equipment even when the plurality of users sleep in the same space. A degree of an effect that a stimulus, such as lighting, has on sleep varies depending on sleep states. For this reason, by taking into consideration a sleep state of the second user when performing control of a stimulus, such a lighting, to the first user, the effect of the stimulus to the first user can be increased within a range where the second user's sleep is not disturbed. Thus, when the first user and the second user sleep in the same space, it is possible to induce the first user to comfortably fall asleep or wake up without disturbing the second user's sleep.

According to the above information processing method, in adjustment of the stimulus control pattern, the acquired stimulus control pattern may be adjusted so that a stimulus given to the first user when the sleep state is lighter than a reference sleep state becomes weaker than a stimulus given to the first user when the sleep state is deeper than the reference sleep state.

According to this configuration, when the sleep state of the second user is lighter than the reference sleep state, a stimulus that does not disturb the second user's sleep is given to the first user. When the first user is induced to fall asleep or wake up, therefore, it is possible to prevent the second user's sleep from being disturbed.

According to the above information processing method, in adjustment of the stimulus control pattern, the acquired stimulus control pattern may be adjusted so that the stimulus becomes weaker than a stimulus determined by the acquired stimulus control pattern.

According to this configuration, the acquired stimulus control pattern is adjusted so that the stimulus becomes weaker than a stimulus determined by the acquired stimulus control pattern. This makes a stimulus given to the second user weaker, thus preventing the second user's sleep from being disturbed.

According to the above information processing method, in adjustment of the stimulus control pattern, the acquired stimulus control pattern may be adjusted so that a point of time of intensifying the stimulus in the acquired stimulus control pattern arrives earlier when the sleep state is deeper than the reference sleep state.

According to this configuration, the point of time of intensifying the stimulus in the acquired stimulus control pattern is caused to arrive earlier when the sleep state of the second user is deeper than the reference sleep state. This makes the sleep state of the first user lighter than the reference sleep state at an earlier point of time, thus allowing the first user to certainly wake up at a scheduled wake-up time.

According to the above information processing method, in adjustment of the stimulus control pattern, an output setting range for the acquired stimulus control pattern may be adjusted.

According to this configuration, the output setting range for the acquired stimulus control pattern is adjusted. As a result, optimum output to induce the first user to comfortably fall asleep or wake up can be set in the output setting range.

According to the above information processing method, a first scheduled wake-up time of the first user and a second scheduled wake-up time of the second user may be acquired, and, in adjustment of the stimulus control pattern, an output of the acquired stimulus control pattern may be adjusted within the output setting range, based on a difference between the first scheduled wake-up time and the second scheduled wake-up time.

According to this configuration, the smaller the difference between the first scheduled wake-up time and the second scheduled wake-up time becomes, the larger the output of the stimulus control pattern is made. This helps the first user in falling asleep or waking up while giving priority to ensuring the comfortability of the first user. Contrary to that, the larger the difference between the first scheduled wake-up time and the second scheduled wake-up time becomes, the smaller the output of the stimulus control pattern is made. This helps the first user in falling asleep or waking up while giving priority to ensuring the comfortability of the second user.

According to the above information processing method, an attribute or preference of at least one of the first user and the second user may be acquired, and, in adjustment of the stimulus control pattern, an output of the acquired stimulus control pattern may be adjusted within the output setting range, based on the acquired attribute or preference.

According to this configuration, for example, the output of the stimulus control pattern for controlling a stimulus given to the first user can be made larger or smaller, based on the attribute of the second user, such as sex and age. In another case, for example, the output of the stimulus control pattern for controlling a stimulus to the first user can be made larger or smaller, based on the first user's preference to a stimulus that is given to the first user when the first user falls asleep or wakes up.

According to the above information processing method, priority level information indicating which of the first user and the second user is to be given priority may be obtained, and, in adjustment of the stimulus control pattern, an output of the acquired stimulus control pattern may be adjusted within the output setting range, based on the acquired priority level information.

According to this configuration, the output of the acquired stimulus control pattern is adjusted within the output setting range, based on the acquired priority level information indicating which of the first user and the second user is to be given priority. When the first user is given priority over the second user, therefore, the output of the acquired stimulus control pattern is increased so as to help the first user in falling asleep or waking up while giving priority to the comfortability of the first user. When the second user is given priority over the first user, in contrast, the output of the acquired stimulus control pattern is reduced so as to help the first user in falling asleep or waking up while giving priority to the comfortability of the second user.

According to the above information processing method, a position of the second user may be acquired, and, in adjustment of the stimulus control pattern, an output of the acquired stimulus control pattern may be adjusted within the output setting range, based on the acquired position.

According to this configuration, the output of the acquired stimulus control pattern is adjusted within the output setting range, based on the position of the second user. Thus, when the second user takes a position in which the second user is apt to be exposed to a stimulus by the one or more devices, for example, the output of the stimulus control pattern is reduced to prevent the second user's sleep from being disturbed.

According to the above information processing method, the one or more devices may include a first device associated with the first user and a second device associated with the second user, and, in control of the operation, the operation of the first device may be controlled according to the adjusted stimulus control pattern.

According to this configuration, the one or more devices include the first device associated with the first user and the second device associated with the second user, and the operation of the first device is controlled according to the adjusted stimulus control pattern. Thus, when the first user is helped in falling asleep or waking up, only the operation of the first device associated with the first use is controlled. As a result, it is possible to more reliably prevent the second user's sleep from being disturbed.

According to the above information processing method, the one or more devices may include at least one of a lighting device, which lights up the space, a sound output device, which outputs a sound into the space, an air freshener diffuser, which diffuses an air freshener in the space, and an airflow output device, which sends airflows into the space.

According to this configuration, the operation of at least one of the lighting device, which lights up the space, the sound output device, which outputs a sound into the space, the air freshener diffuser, which diffuses an air freshener in the space, and the airflow output device, which sends airflows into the space, is controlled. Thus, at least one of light, a sound, a fragrance of the air freshener, and an airflow induces the first user to comfortably fall asleep or wake up.

An information processing program according to another aspect of the present disclosure causes a computer to: acquire a stimulus control pattern for controlling a stimulus that one or more devices give to the first user to induce the first user to fall asleep or wake up; acquire a sleep state of a second user who is in the same space in which the first user is; adjust the acquired stimulus control pattern, based on the sleep state of the second user; and control an operation of the one or more devices, according to the adjusted stimulus control pattern.

In this configuration, the stimulus control pattern is adjusted based on the sleep state of the second user who is in the same space in which the first user is, and the operation of the one or more devices is controlled according to the adjusted stimulus control pattern. As a result, a reduction in an effect of facilitating falling asleep or waking up, the reduction being caused by control of a stimulus, such as lighting, to a specific user (i.e., the first user) among a plurality of users, can be inhibited without using physical equipment even when the plurality of users sleep in the same space. A degree of an effect that a stimulus, such as lighting, has on sleep varies depending on sleep states. For this reason, by taking into consideration a sleep state of the second user when performing control of a stimulus, such a lighting, to the first user, the effect of the stimulus to the first user can be increased within a range where the second user's sleep is not disturbed. Thus, when the first user and the second user sleep in the same space, it is possible to induce the first user to comfortably fall asleep or wake up without disturbing the second user's sleep.

An information processing system according to another aspect of the present disclosure includes: a stimulus control pattern acquiring unit that acquires a stimulus control pattern for controlling a stimulus that one or more devices give to a first user to induce the first user to fall asleep or wake up; a sleep state acquiring unit that acquires a sleep state of a second user who is in the same space in which the first user is; an adjusting unit that adjusts the acquired stimulus control pattern, based on the sleep state of the second user; and a control unit that controls an operation of the one or more devices, according to the adjusted stimulus control pattern.

In this configuration, the stimulus control pattern is adjusted based on the sleep state of the second user who is in the same space in which the first user is, and the operation of the one or more devices is controlled according to the adjusted stimulus control pattern. As a result, a reduction in an effect of facilitating falling asleep or waking up, the reduction being caused by control of a stimulus, such as lighting, to a specific user (i.e., the first user) among a plurality of users, can be inhibited without using physical equipment even when the plurality of users sleep in the same space. A degree of an effect that a stimulus, such as lighting, has on sleep varies depending on sleep states. For this reason, by taking into consideration a sleep state of the second user when performing control of a stimulus, such a lighting, to the first user, the effect of the stimulus to the first user can be increased within a range where the second user's sleep is not disturbed. Thus, when the first user and the second user sleep in the same space, it is possible to induce the first user to comfortably fall asleep or wake up without disturbing the second user's sleep.

Embodiments of the present disclosure will hereinafter be described with reference to the drawings. The following embodiments are examples for embodying the present disclosure and do not put limits on the technical scope of the present disclosure.

### (First Embodiment)

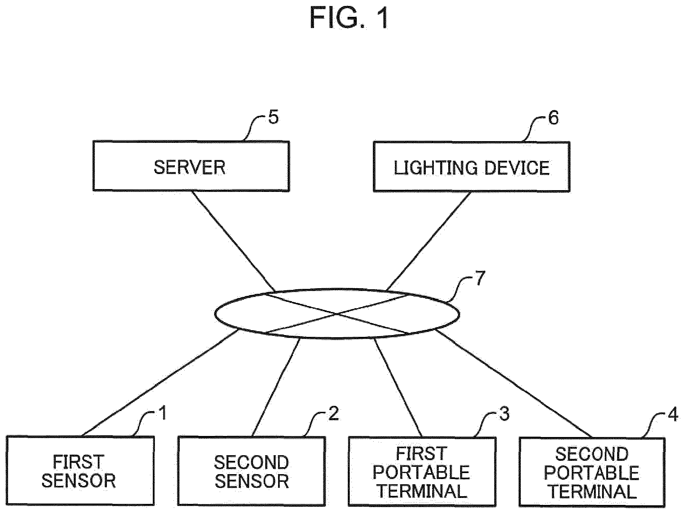

FIG. 1 depicts an overall configuration of an equipment control system according to a first embodiment of the present disclosure.

The equipment control system shown in FIG. 1 includes a first sensor 1, a second sensor 2, a first portable terminal 3, a second portable terminal 4, a server 5, and a lighting device 6. The server 5 is connected to the first sensor 1, the second sensor 2, the first portable terminal 3, the second portable terminal 4, and the lighting device 6, respectively, via a network 7 so that the server 5 can communicate with them. The network 7 is provided as, for example, the Internet.

The first sensor 1 is provided as, for example, a sheetlike piezoelectric sensor placed under a bed mat on which a first user lies. The first sensor 1 acquires biometric data on the first user, such as the first user's heart rate, breathing rate, and body motion. The first sensor 1 acquires the biometric data on the first user periodically (e.g., every 10 minutes). The first sensor 1 sends the acquired biometric data on the first user, to the server 5.

The second sensor 2 is provided as, for example, a sheetlike piezoelectric sensor placed under a bed mat on which a second user lies. The second sensor 2 acquires biometric data on the second user, such as the second user's heart rate, breathing rate, and body motion. The second sensor 2 acquires the biometric data on the second user periodically (e.g., every 10 minutes). The second sensor 2 sends the acquired biometric data on the second user, to the server 5.

Each of the first sensor 1 and the second sensor 2 may be provided as a sensor different from the piezoelectric sensor, such as an electric wave sensor that acquires biometric data by a method different from a method used by the piezoelectric sensor. The equipment control system of the first embodiment includes the first sensor 1 and the second sensor 2. According to the present disclosure, however, the configuration of the equipment control system is not limited to this. The equipment control system may include a single sensor that acquires biometric data on the first user and biometric data on the second user as well.

When the first user is lying on the bed mat, the first sensor 1 acquires the biometric data on the first user. When the first user is not lying on the bed mat, therefore, the first sensor 1 does not acquire the biometric data on the first user, thus sending no biometric data on the first user to the server 5. The second sensor 2 does the same in acquisition and transmission of the biometric data on the second user.

The first portable terminal 3 is provided as, for example, a smart phone, a tablet computer, or a personal computer, and is used by the first user. The first portable terminal 3 receives input of a scheduled wake-up time of the first user. The first portable terminal 3 sends the scheduled wake-up time of the first user to the server 5.

The second portable terminal 4 is provided as, for example, a smart phone, a tablet computer, or a personal computer, and is used by the second user. The second portable terminal 4 receives input of a scheduled wake-up time of the second user. The second portable terminal 4 sends the scheduled wake-up time of the second user to the server 5.

The equipment control system of the first embodiment includes the first portable terminal 3 and the second portable terminal 4. According to the present disclosure, however, the configuration of the equipment control system is not limited to this. The equipment control system may include a single portable terminal that is used by both the first user and the second user and that receives a scheduled wake-up time of the first user and a scheduled wake-up time of the second user as well.

The server 5 controls the operation of the lighting device 6 that controls illuminance for helping the first user or the second user in waking up. The server 5 sends a control command for controlling the operation of the lighting device 6, to the lighting device 6.

The lighting device 6 is disposed in a space in which the first user and the second user sleep, that is, a bedroom. The lighting device 6 lights up the bedroom interior and is capable of adjusting illuminance. The lighting device 6 controls the illuminance for helping the first user or the second user in waking up. The lighting device 6 controls the illuminance according to a control command from the server 5. The lighting device 6 is an example of one or more devices that control a stimulus for helping the first user in falling asleep or waking up.

According to the first embodiment, one lighting device 6 is disposed in the bedroom. The lighting device 6 is used by both the first user and the second user.

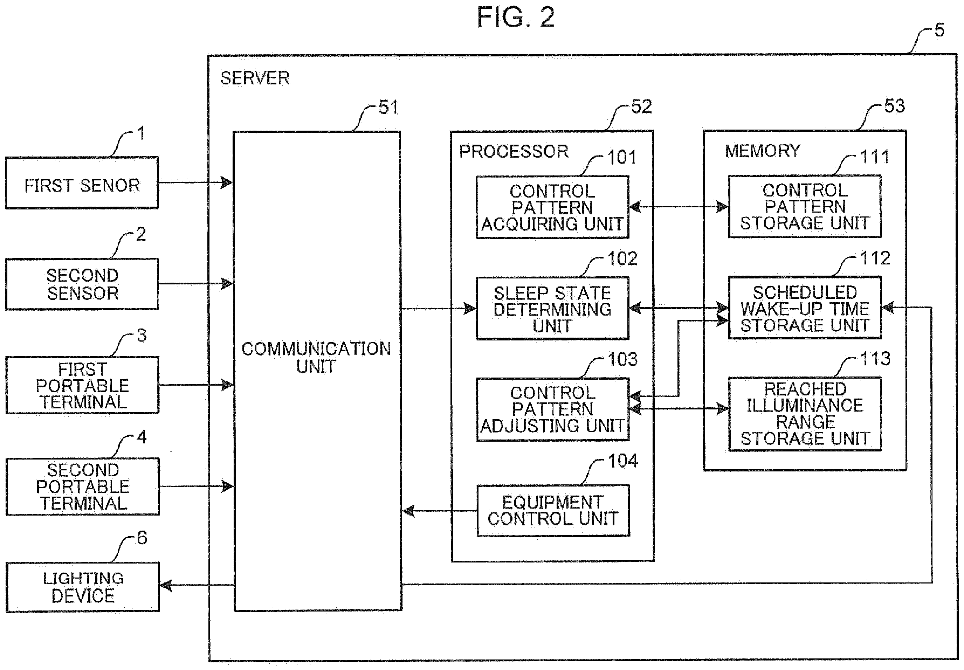

FIG. 2 depicts a configuration of the server according to the first embodiment of the present disclosure.

The server 5 includes a communication unit 51, a processor 52, and a memory 53. The processor 52 has a control pattern acquiring unit 101, a sleep state determining unit 102, a control pattern adjusting unit 103, and an equipment control unit 104. The memory 53 has a control pattern storage unit 111, a scheduled wake-up time storage unit 112, and a reached illuminance range storage unit 113.

The communication unit 51 receives biometric data on the first user, the biometric data being sent by the first sensor 1. The communication unit 51 outputs the received biometric data on the first user, to the sleep state determining unit 102. The communication unit 51 receives biometric data on the second user, the biometric data being sent by the second sensor 2. The communication unit 51 outputs the received biometric data on the second user, to the sleep state determining unit 102. The communication unit 51 receives a scheduled wake-up time of the first user sent by the first portable terminal 3. The communication unit 51 stores the received scheduled wake-up time of the first user in the scheduled wake-up time storage unit 112. The communication unit 51 receives a scheduled wake-up time of the second user sent by the second portable terminal 4. The communication unit 51 stores the received scheduled wake-up time of the second user in the scheduled wake-up time storage unit 112. The communication unit 51 sends a control command generated by the equipment control unit 104, to the lighting device 6.

The control pattern storage unit 111 stores therein a stimulus control pattern for controlling an operation of the lighting device 6 that is carried out when the user wakes up. According to the stimulus control pattern, the lighting device 6 operates in such a way as to gradually increase the illuminance from a first point of time a given time (e.g., 30 minutes) prior to a scheduled wake-up time, to sharply raise the illuminance at a second point of time which is a given time (e.g., 3 minutes) prior to the scheduled wake-up time, that is, right before the scheduled wake-up time, and to raise the illuminance to its maximum at the scheduled wake-up time. The above stimulus control pattern is an example. According to the present disclosure, a different stimulus control pattern may be adopted to help the first user in waking up comfortably.

The scheduled wake-up time storage unit 112 stores the scheduled wake-up time of the first user and the scheduled wake-up time of the second user.

The reached illuminance range storage unit 113 stores a table in which a sleep state of the second user who wakes up later is associated with a reached illuminance setting range indicating an upper limit and a lower limit of a reached illuminance, which should be reached at the scheduled wake-up time of the first user who wakes up first, for each type of the lighting device.

The control pattern acquiring unit 101 acquires a stimulus control pattern for controlling a stimulus that the lighting device 6 gives to the first user to induce the first user to wake up.

The sleep state determining unit 102 refers to the scheduled wake-up time storage unit 112, identifies the user who wakes up later out of the first and second users, and determines a sleep state of the identified user who wakes up later.

When the second user wakes up later, the sleep state determining unit 102 determines a sleep state of the second user, based on biometric data on the second user, the biometric data being acquired from the second sensor 2.

A person's state of sleeping can be classified into multiple sleep states that shift as time goes by, according to the deepness and characteristics of sleep. Generally, these sleep states are classified into REM sleep and non-REM sleep. REM sleep is a type of sleep that accompanies high-speed eye movement. In a state of REM sleep, the person's body is at rest but brain is active. It is reported that, in many cases, people dream during their REM sleep. Non-REM sleep is a type of sleep that accompanies no high-speed eye movement. Non-REM sleep is further classified into four stages, i.e., stage 1 to stage 4 according to the deepness of sleep. The stage 4 represents a state of the deepest sleep. Usually, after a person falls asleep, the person's sleep reaches the stage 3 or 4 of non-REM sleep within a period of 45 to 60 minutes, and then becomes light gradually during an ensuing period of 1 to 2 hours to shift to REM sleep. Afterward, non-REM sleep and REM sleep appear repeatedly by turns in 90 to 110 minutes (or 80 to 100 minutes) sleep cycles. Individuals' sleeping behavior, however, vary greatly depending on their personal variations and differences.

Biometric data, such as body motions, a breathing rate, and a heart rate, has a correlation with a sleep state. For example, it is known that in a deep sleep state, such as the stage 3 or 4 of non-REM sleep, the person's body motions are few and heart rate variability (R-R interval or RRI) is low. Using such a correlation, the sleep state determining unit 102 infers a sleep state of the user from the biometric data in a real-time manner. Based on the biometric data, the sleep state determining unit 102 determines which of "awaked", "REM sleep", "stage 1 of non-REM sleep", "stage 2 of non-REM sleep", "stage 3 of non-REM sleep", and "stage 4 of non-REM sleep" corresponds to the sleep state of the user.

Sleep states include an awaked state, a light sleep state, and a deep sleep state. The awaked state refers to a state in which the user has awaked from sleep. The light sleep state refers to a state in which the user is in the stage 1 of non-REM sleep or the stage 2 of non-REM sleep. The deep sleep state refers to a state in which the user is in the stage 3 of non-REM sleep or the stage 4 of non-REM sleep. It is said that REM sleep is similar to the stage 1 in terms of brain wave types. REM sleep is thus often regarded as a state of light sleep. In REM sleep, however, muscular depression is at work to suppress the frontal lobe's activities, leading to a conclusion that REM sleep represents a sleep state from which awaking is so difficult that it cannot be referred to as light sleep. For this reasons, REM sleep is defined here as a deep sleep state. The sleep state determining unit 102 determines whether a sleep state of the second user is the awaked state, the light sleep state, or the deep sleep state.

When the first user wakes up later, the sleep state determining unit 102 determines a sleep state of the first user, based on biometric data on the first user, the biometric data being acquired from the first sensor 1. In this case, the sleep state determining unit 102 determines whether the sleep state of the first user is the awaked state, the light sleep state, or the deep sleep state.

According to the first embodiment, the stage 1 of non-REM sleep or the stage 2 of non-REM sleep is equivalent to the light sleep state, while the stage 3 of non-REM sleep or the stage 4 of non-REM sleep is equivalent to the deep sleep state. Sleep state definition of the present disclosure, however, is not limited to this. For example, sleep states may be defined such that the stage 1 of non-REM sleep is equivalent to the light sleep state, while the stage 2 of non-REM sleep, the stage 3 of non-REM sleep, or the stage 4 of non-REM sleep is equivalent to the deep sleep state. Sleep states may also be defined differently such that the stage 1 of non-REM sleep, the stage 2 of non-REM sleep, and the stage 3 of non-REM sleep are each equivalent to the light sleep state, while the stage 4 of non-REM sleep is equivalent to the deep sleep state.

The control pattern adjusting unit 103 acquires a sleep state of the second user who is in the same space in which the first user is. The first user wakes up earlier than the second user. The control pattern adjusting unit 103, therefore, acquires the sleep state of the second user, who wakes up later, from the sleep state determining unit 102.

According to the first embodiment, the sleep state determining unit 102 refers to the scheduled wake-up time storage unit 112, identifies the user who wakes up later out of the first and second users, and determines a sleep state of the identified user. The process of acquiring the sleep state of the identified user according to the present disclosure, however, is not limited to this. The process of acquiring the sleep state of the identified user may be carried out such that the sleep state determining unit 102 determines a sleep state of the first user and a sleep state of the second user as well, and the control pattern adjusting unit 103 refers to the scheduled wake-up time storage unit 112, identifies the user who wakes up later out of the first and second users, and acquires a sleep state of the identified user from the sleep state determining unit 102.

Based on the sleep state of the second user, the control pattern adjusting unit 103 adjusts a stimulus control pattern acquired by the control pattern acquiring unit 101. The control pattern adjusting unit 103 adjusts the acquired stimulus control pattern so that a stimulus given to the first user when the sleep state of the second user is lighter than a reference sleep state becomes weaker than a stimulus given to the first user when the sleep state of the second user is deeper than the reference sleep state. According to the first embodiment, the stimulus given to the first user is light from a lighting device. The control pattern adjusting unit 103 adjusts the acquired stimulus control pattern so that the stimulus becomes weaker than a stimulus determined by the acquired stimulus control pattern.

The control pattern adjusting unit 103 adjusts a reached illuminance setting range (output setting range) for a stimulus control pattern acquired by the control pattern acquiring unit 101. The control pattern adjusting unit 103 refers to the reached illuminance range storage unit 113, and determines a reached illuminance setting range according to a sleep state of the second user determined by the sleep state determining unit 102.

The control pattern adjusting unit 103 acquires a scheduled wake-up time of the first user (first scheduled wake-up time) and a scheduled wake-up time of the second user (second scheduled wake-up time), from the scheduled wake-up time storage unit 112. Based on a difference between the scheduled wake-up time of the first user (first scheduled wake-up time) and the scheduled wake-up time of the second user (second scheduled wake-up time), the control pattern adjusting unit 103 adjusts a reached illuminance (output) of the stimulus control pattern, within the reached illuminance setting range (output setting range). For example, the control pattern adjusting unit 103 adjusts the reached illuminance to a reached illuminance value closer to an upper limit of the reached illuminance setting range when the difference between the scheduled wake-up time of the first user and the scheduled wake-up time of the second user is shorter, while adjusts the reached illuminance to a reached illuminance value closer to a lower limit of the reached illuminance setting range when the difference between the scheduled wake-up time of the first user and the scheduled wake-up time of the second user is longer.

The case where the difference between the scheduled wake-up time of the first user and the scheduled wake-up time of the second user is short refers to a case where a timespan between a point of time of the first user's waking up and a point of time of second user's waking up later is short. In this case, even if the second user is exposed to high reached illuminance, which is a stimulus to the first user for helping the first user in waking up, and wakes up because of the high illuminance, it does not pose any particular problem because the scheduled wake-up time of the second user is close to the scheduled wake-up time of the first user. The case where the difference between the scheduled wake-up time of the first user and the scheduled wake-up time of the second user is long refers to a case where a timespan between a point of time of the first user's waking up and a point of time of second user's waking up later is long. In this case, the reached illuminance for helping the first user in waking up is kept low. This prevents the second user from waking up.

The equipment control unit 104 controls the operation of the lighting device 6 according to a stimulus control pattern adjusted by the control pattern adjusting unit 103. The equipment control unit 104 generates a control command for causing the lighting device 6 to operate according to the stimulus control pattern adjusted by the control pattern adjusting unit 103.

The lighting device 6 has a light source (not depicted), and operates according to an incoming control command from the server 5. The lighting device 6 operates according to a stimulus control pattern for helping the first user in waking up.

According to the first embodiment, the first portable terminal 3 or second portable terminal 4 may have the communication unit 51, the processor 52, and the memory 53, which are respective components of the server 5. In this case, the server 5 is unnecessary.

An example of the first embodiment will hereinafter be described. In this example, the lighting device 6 is a single ceiling light installed in a main bedroom, the first user wakes up earlier than the second user, and light control is performed by the lighting device 6 to control light emitted onto the first user to be woken up.

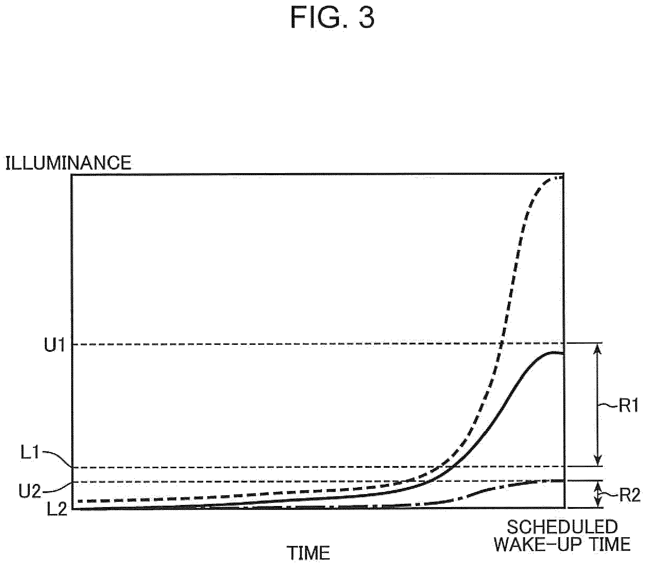

FIG. 3 depicts an example of stimulus control patterns for controlling a stimulus given to the first user who wakes up first, according to first embodiment of the present disclosure. In FIG. 3, the vertical axis represents illuminance while the horizontal axis represents time. In FIG. 3, a broken line represents an ordinary reference stimulus control pattern in a case where one person is asleep in bed. According to the reference stimulus control pattern, light control is carried out in such a way as to gradually increase illuminance from a point of time a given time (e.g., 30 minutes) prior to a scheduled wake-up time and to sharply raise the illuminance to its maximum in a period between a point of time a given time (e.g., 3 minutes) prior to the scheduled wake-up time, that is, right before the scheduled wake-up time and the scheduled wake-up time. Through this control, an optical stimulus given to the first user facilitates the first user's waking up with a refreshed feeling.

According to the first embodiment, the illuminance increase is started at arrival of the preset point of time the given time before the scheduled wake-up time. The illuminance increase pattern according to the present disclosure, however, is not limited to this. For example, when a preset point of time a given time (e.g., 1 hour) before the scheduled wake-up time arrives, whether the sleep state of the first user is the deep sleep state may be determined. When it is determined that the sleep state of the first user is the deep sleep state, the illuminance increase may be started. When it is determined that the sleep state of the first user is not the deep sleep state, on the other hand, the illuminance increase may not be started. Afterward, at a point of time at which the first user's sleep state being the deep sleep state is determined, the illuminance increase may be started.

According to the first embodiment, the illuminance is raised sharply at the point of time the given time prior to the scheduled wake-up time, that is, right before the scheduled wake-up time, to reach the maximum. The illuminance increase pattern according to the present disclosure, however, is not limited to this. For example, whether the sleep state of the first user is the light sleep state may be determined. When it is determined that the sleep state of the first user is the light sleep state, the illuminance may be raised sharply to the maximum. When it is determined that the sleep state of the first user is not the light sleep state, on the other hand, the illuminance may not be raised sharply but may be raised gradually.

Based on a sleep state of the second user determined by the sleep state determining unit 102, the control pattern adjusting unit 103 adjusts the reference stimulus control pattern indicated by the broken line. Specifically, the control pattern adjusting unit 103 sets a reached illuminance at the scheduled wake-up time that is lower than a reached illuminance of a normal level (maximum).

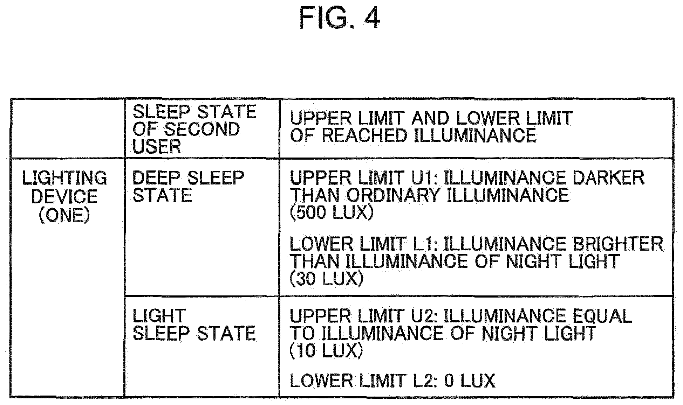

FIG. 4 depicts an example of a table stored in the reached illuminance range storage unit according to the first embodiment of the present disclosure.

The control pattern adjusting unit 103 refers to the table of FIG. 4 stored in the reached illuminance range storage unit 113, determines a reached illuminance setting range for the reached illuminance of the stimulus control pattern, according to the sleep state of the second user, and determines a reached illuminance within the determined reached illuminance setting range.

As shown in FIG. 4, the reached illuminance range storage unit 113 stores a table in which a sleep state of the second user who wakes up later is associated with a reached illuminance setting range indicating an upper limit U1 and a lower limit L1 of a reached illuminance, which should be reached at the scheduled wake-up time of the first user who wakes up first, for each type of the lighting device. According to the first embodiment, only one lighting device 6 is disposed in the main bedroom.

The upper limit U1 representing an illuminance (e.g., 500 lux) darker than an ordinary illuminance and the lower limit L1 representing an illuminance (e.g., 30 lux) brighter than the illuminance of a night light are associated with the deep sleep state. The ordinary illuminance is equivalent to the maximum illuminance of the lighting device 6, which is, for example, 1000 lux. The illuminance of a night light is, for example, 10 lux. The upper limit U1 represents an illuminance that is, for example, the half of the ordinary illuminance.

An upper limit U2 representing an illuminance (e.g., 10 lux) equal to the illuminance of a night light and a lower limit L2 representing 0 lux are associated with the light sleep state.

In FIG. 3, a continuous line indicates a stimulus control pattern for a case where the sleep state of the second user is the deep sleep state, and a single-dot chain line indicates a stimulus control pattern for a case where the sleep state of the second user is the light sleep state. A range between the upper limit U1 and the lower limit L1 of the reached illuminance is a reached illuminance setting range R1, while a range between the upper limit U2 and the lower limit L2 of the reached illuminance is a reached illuminance setting range R2.

As shown in FIG. 3, the reached illuminance of the stimulus control pattern for the case where the sleep state of the second user is the deep sleep state is smaller than the reached illuminance of the reference stimulus control pattern. The reached illuminance of the stimulus control pattern for the case where the sleep state of the second user is the light sleep state is smaller than the reached illuminance of the stimulus control pattern for the case where the sleep state of the second user is the deep sleep state.

According to the first embodiment, the equipment control system includes one lighting device 6 attached to the ceiling of the bedroom. The configuration of the equipment control system according to the present disclosure, however, is not limited to this. The equipment control system may include a first lighting device that separately lights up the first user and a second lighting device that separately lights up the second user.

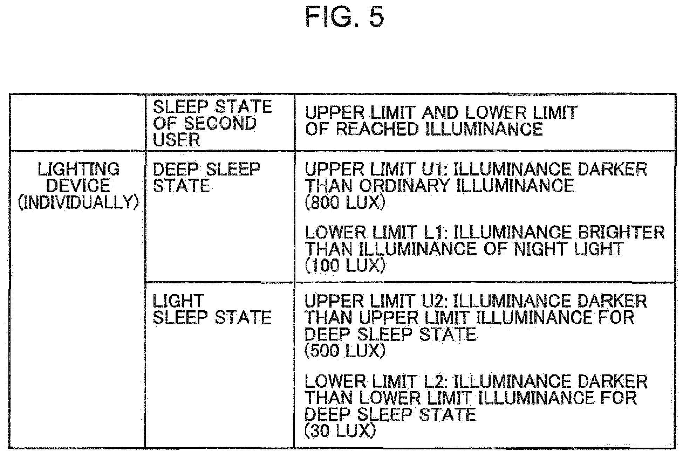

FIG. 5 depicts an example of a table that is stored in the reached illuminance range storage unit when the first user and the second user are separately lighted up, respectively, according to the first embodiment of the present disclosure.

As shown in FIG. 5, the reached illuminance range storage unit 113 stores a table in which a sleep state of the second user who wakes up later is associated with a reached illuminance setting range indicating an upper limit U1 and a lower limit L1 of a reached illuminance, which should be reached at the scheduled wake-up time of the first user who wakes up first, for each type of the lighting device. The lighting device includes the first lighting device associated with the first user, and the second lighting device associated with the second user.

The equipment control unit 104 controls the operation of the first lighting device according to a stimulus control pattern adjusted by the control pattern adjusting unit 103. The equipment control unit 104 generates a control command for causing the first lighting device to operate according to the stimulus control pattern adjusted by the control pattern adjusting unit 103. The first lighting device operates according to an incoming control command from the server 5. The first lighting device operates according to a stimulus control pattern for helping the first user in waking up.

The upper limit U1 and the lower limit L1 are associated with the deep sleep state, the upper limit U1 representing an illuminance (e.g., 800 lux) darker than the ordinary illuminance and the lower limit L1 representing an illuminance (e.g., 100 lux) brighter than the illuminance level of a night light. The ordinary illuminance is equivalent to the maximum illuminance of the first lighting device, which is, for example, 1000 lux. The illuminance of a night light is, for example, 10 lux. The upper limit U1 represents an illuminance level that is, for example, 80% of the ordinary illuminance.

An upper limit U2 and a lower limit L2 are associated with the light sleep state, the upper limit U2 representing an illuminance (e.g., 500 lux) darker than the illuminance indicated by the upper limit U1 for the deep sleep state, and the lower limit L2 representing an illuminance (e.g., 30 lux) darker than the illuminance indicated by the lower limit L1 for the deep sleep state.

The first lighting device lights up the first user only. For this reason, the upper limit and lower limit of the reached illuminance of each of the first lighting device and the second lighting device, which are used for the first user and the second user, respectively, are determined to be higher respectively than the upper limit and lower limit of the reached illuminance of the one lighting device used in common for both the first user and the second user.

According to the first embodiment, the equipment control system may include a first lighting device that separately lights up the first user's eyes and a second lighting device that separately lights up the second user's eyes.

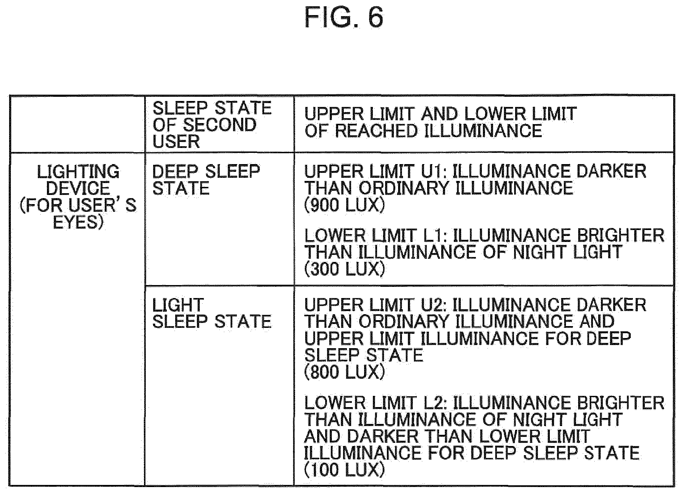

FIG. 6 depicts an example of a table that is stored in the reached illuminance range storage unit when the first user's eyes and the second user's eyes are separately lighted up, respectively, according to the first embodiment of the present disclosure.

As shown in FIG. 6, the reached illuminance range storage unit 113 stores a table in which a sleep state of the second user who wakes up later is associated with a reached illuminance setting range indicating an upper limit U1 and a lower limit L1 of a reached illuminance, which should be reached at the scheduled wake-up time of the first user who wakes up first, for each type of the lighting device. The lighting device includes the first lighting device that lights up the first user's eyes, and the second lighting device that lights up the second user's eyes.

The equipment control unit 104 controls the operation of the first lighting device according to a stimulus control pattern adjusted by the control pattern adjusting unit 103. The equipment control unit 104 generates a control command for causing the first lighting device to operate according to the stimulus control pattern adjusted by the control pattern adjusting unit 103. The first lighting device operates according to an incoming control command from the server 5. The first lighting device operates according to a stimulus control pattern for helping the first user in waking up.

The upper limit U1 and the lower limit L1 are associated with the deep sleep state, the upper limit U1 representing an illuminance (e.g., 900 lux) darker than the ordinary illuminance and the lower limit L1 representing an illuminance (e.g., 300 lux) brighter than the illuminance of a night light. The ordinary illuminance is equivalent to the maximum illuminance of the first lighting device, which is, for example, 1000 lux. The illuminance of a night light is, for example, 10 lux. The upper limit U1 represents an illuminance that is, for example, 90% of the ordinary illuminance.

An upper limit U2 and a lower limit L2 are associated with the light sleep state, the upper limit U2 representing an illuminance (e.g., 800 lux) that is darker than the ordinary illuminance and than the illuminance indicated by the upper limit U1 for the deep sleep state and the lower limit L2 representing an illuminance (e.g., 100 lux) that is brighter than the illuminance of a night light but is darker than the illuminance indicated by the lower limit L1 for the deep sleep state.

The first lighting device lights up the first user's eyes only. For this reason, the upper limit and lower limit of the reached illuminance of each of the first lighting device and the second lighting device, which light up the first user's eyes and the second user's eyes, respectively, are higher respectively than the upper limit and lower limit of the reached illuminance of the one lighting device used in common for both the first user and the second user and are also higher respectively than the upper limit and lower limit of the reached illuminance of each of the first lighting device and the second lighting device used separately for the first user and the second user, respectively.

In this manner, the upper limit and the lower limit of the reached illuminance may be determined in accordance with the type of the lighting device used in each home.

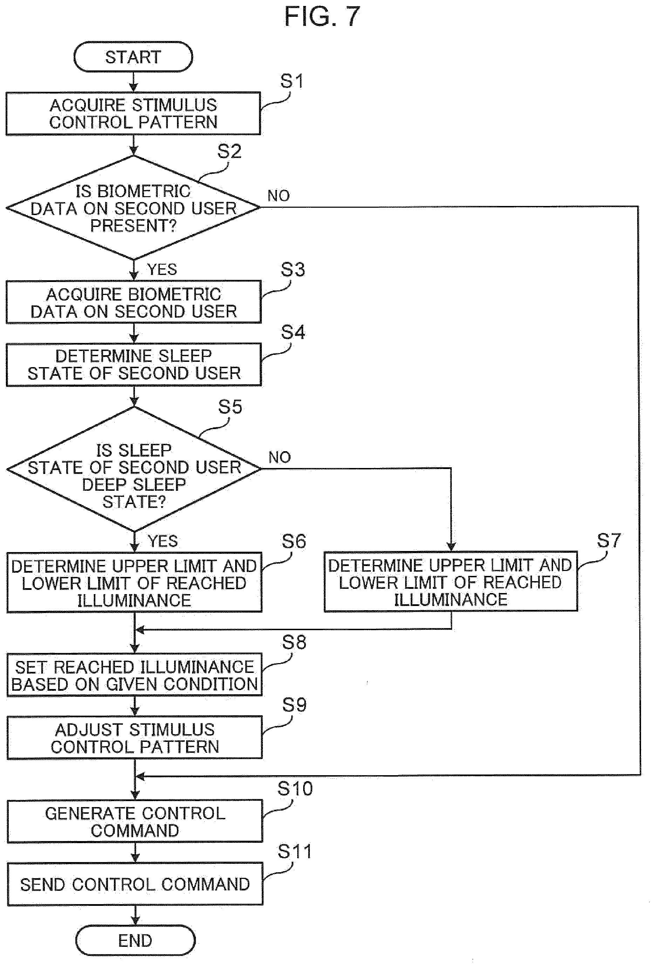

FIG. 7 is a flowchart for explaining an operation of the server according to the first embodiment of the present disclosure.

When a point of time given time (e.g., 30 minutes) prior to the scheduled wake-up time of the first user, who wakes up earlier than the second user, arrives, the control pattern acquiring unit 101 acquires a stimulus control pattern for controlling a stimulus that the lighting device 6 gives to the first user to induce the first user to wake up, from the control pattern storage unit 111 (step S1). The control pattern acquiring unit 101 refers to the scheduled wake-up time storage unit 112, thereby acquires the scheduled wake-up time of the first user, who wakes up earlier than the second user.

Subsequently, the sleep state determining unit 102 determines whether biometric data on the second user, who wakes up after the first user wakes up, is present (step S2). When the second user is not asleep in bed, the biometric data on the second user is not detected. By determining whether the biometric data on the second user is present, therefore, whether the second user is asleep in bed can be determined.

When it is determined that the biometric data on the second user is not present (NO at step S2), the process flow proceeds to step S11.

When it is determined that the biometric data on the second user is present (YES at step S2), on the other hand, the sleep state determining unit 102 acquires the biometric data on the second user detected by the second sensor 2 (step S3).

The sleep state determining unit 102 then determines a sleep state of the second user, based on the acquired biometric data on the second user (step S4).

Subsequently, the control pattern adjusting unit 103 determines whether the sleep state of the second user, which is determined by the sleep state determining unit 102, is the deep sleep state (step S5).

When it is determined that the sleep state of the second user is the deep sleep state (YES at step S5), the control pattern adjusting unit 103 refers to the table stored in the reached illuminance range storage unit 113 and determines an upper limit and a lower limit of a reached illuminance of a stimulus control pattern for the first user, the upper limit and lower limit being associated with the deep sleep state (step S6). When using the table shown in FIG. 4, the control pattern adjusting unit 103 determines the upper limit of the reached illuminance to be 500 lux and determines the lower limit of the same to be 30 lux.

When it is determined that the sleep state of the second user is not the deep sleep state, that is, it is determined that the sleep state of the second user is the light sleep state (NO at step S5), the control pattern adjusting unit 103 refers to the table stored in the reached illuminance range storage unit 113 and determines an upper limit and a lower limit of a reached illuminance of a stimulus control pattern for the first user, the upper limit and lower limit being associated with the light sleep state (step S7). When using the table shown in FIG. 4, the control pattern adjusting unit 103 determines the upper limit of the reached illuminance to be 10 lux and determines the lower limit of the same to be 0 lux.

Based on a given condition, the control pattern adjusting unit 103 then sets a reached illuminance within a reached illuminance setting range between the determined upper limit and the lower limit (step S8). According to the first embodiment, the given condition is the length of a difference between the scheduled wake-up time of the first user and the scheduled wake-up time of the second user. The control pattern adjusting unit 103 sets a reached illuminance closer to the upper limit in the reached illuminance setting range when the difference between the scheduled wake-up time of the first user and the scheduled wake-up time of the second user is shorter, while sets a reached illuminance closer to the lower limit in the reached illuminance setting range when the difference between the scheduled wake-up time of the first user and the scheduled wake-up time of the second user is longer.

For example, the control pattern adjusting unit 103 may set the reached illuminance matching the upper limit when the difference between the scheduled wake-up time of the first user and the scheduled wake-up time of the second user is 5 minutes or shorter, and may set the reached illuminance matching the lower limit when the difference between the scheduled wake-up time of the first user and the scheduled wake-up time of the second user is 1 hour or longer. Further, when the difference between the scheduled wake-up time of the first user and the scheduled wake-up time of the second user ranges from a time difference longer than 5 minutes to a time difference shorter than 1 hour, the control pattern adjusting unit 103 may set a reached illuminance corresponding to a time difference in the reached illuminance setting range.

The control pattern adjusting unit 103 may determine whether the difference between the scheduled wake-up time of the first user and the scheduled wake-up time of the second user is shorter than a given time (e.g., 30 minutes). When determining that the difference between the scheduled wake-up time of the first user and the scheduled wake-up time of the second user is shorter than the given time, the control pattern adjusting unit 103 may set the reached illuminance matching the upper limit. When determining that the difference between the scheduled wake-up time of the first user and the scheduled wake-up time of the second user is equal to or longer than the given time, the control pattern adjusting unit 103 may set the reached illuminance matching the lower limit.

Subsequently, the control pattern adjusting unit 103 adjusts a stimulus control pattern based on a set reached illuminance (step S9). Specifically, the control pattern adjusting unit 103 adjusts the stimulus control pattern so that the illuminance of the lighting device 6 matches the set reached illuminance at the scheduled wake-up time of the first user.

Subsequently, the equipment control unit 104 generates a control command for causing the lighting device 6 to operate according to the stimulus control pattern (step S10). When it is determined that the biometric data on the second user is present and the stimulus control pattern is adjusted by the control pattern adjusting unit 103, the equipment control unit 104 generates a control command for causing the lighting device 6 to operate according to the stimulus control pattern adjusted by the control pattern adjusting unit 103. When it is determined that the biometric data on the second user is not present (NO at step S2), on the other hand, the equipment control unit 104 generates a control command for causing the lighting device 6 to operate according to a stimulus control pattern acquired from the control pattern storage unit 111.

Subsequently, the communication unit 51 sends the control command generated by the equipment control unit 104, to the lighting device 6 (step S11). Receiving the control command, the lighting device 6 operates according to a stimulus control pattern indicated by the received control command.

Thus, when the first user and the second user sleep in the same space, it is possible to induce the first user, who wakes up first, to comfortably wake up without disturbing the second user's sleep, the second user waking up later.

According to the first embodiment, after the control command is sent to the lighting device 6, the lighting device 6 operates according to the stimulus control pattern indicated by the control command until the scheduled wake-up time of the first user arrives. The process flow according to the present disclosure, however, is not limited to this. After the control command is sent to the lighting device 6, for example, processes of steps S1 to S11 shown in FIG. 7 may be executed at every given time. In such a case, even if the sleep state of the second user has changed before arrival of the scheduled wake-up time of the first user, the stimulus control pattern can be adjusted to be applicable to the changed sleep state of the second user.

After the control command is sent to the lighting device 6, the sleep state determining unit 102 may determine whether the sleep state of the second user has changed. When it is determined that the sleep state of the second user has changed, processes of steps S5 to S11 shown in FIG. 7 may be executed. In such a case, even if the sleep state of the second user has changed before arrival of the scheduled wake-up time of the first user, the stimulus control pattern can be adjusted to be applicable to the changed sleep state of the second user.

According to the first embodiment, the control pattern adjusting unit 103 determines the upper limit and the lower limit of the reached illuminance of the stimulus control pattern in accordance with the sleep state of the second user, and sets the reached illuminance within the reached illuminance setting range between the determined upper limit and the lower limit, based on the given condition. The method of setting the reached illuminance according to the present disclosure, however, is not limited to this. For example, the control pattern adjusting unit 103 may set the reached illuminance of the stimulus control pattern just in accordance with the sleep state of the second user. For example, the control pattern adjusting unit 103 may set the reached illuminance as a first reached illuminance darker than the ordinary illuminance when the sleep state of the second user is the deep sleep state, while set the reached illuminance as a second reached illuminance darker than the first reached illuminance when the sleep state of the second user is the light sleep state.

According to the first embodiment, the control pattern adjusting unit 103 sets the reached illuminance within the reached illuminance setting range between the determined upper limit and the lower limit, based on the difference between the scheduled wake-up time of the first user and the scheduled wake-up time of the second user. The method of setting the reached illuminance according to the present disclosure, however, is not limited to this. For example, the control pattern adjusting unit 103 may acquire an attribute of at least one of the first user and the second user. Based on the attribute of at least one of the first user and the second user, the control pattern adjusting unit 103 may set the reached illuminance within the reached illuminance setting range between the determined upper limit and the lower limit.

The attribute is, for example, age or sex. A child or elderly person has low sensitivity to an optical stimulus, and therefore tends to show difficulty in waking up even if exposed to a relatively high reached illuminance. To deal with such a case, when the age of the second user, who wakes up later, is equal to or lower than a given first age (e.g., 15 year-old), that is, when the second user is a child, the control pattern adjusting unit 103 may set the reached illuminance matching the upper limit. In another case where the age of the second user is equal to or higher than a given second age (e.g., 60 year-old), that is, the second user is an elderly person, the control pattern adjusting unit 103 may set the reached illuminance matching the upper limit. When the age of the second user is higher than the first age and lower than the second age, the control pattern adjusting unit 103 may set a reached illuminance corresponding to the age of the second user within the reached illuminance setting range. When the second user is an infant, the control pattern adjusting unit 103 may set the reached illuminance matching the lower limit so that the illuminance does not cause the second user to wake up.

When the second user, who wakes up later, is a male, the control pattern adjusting unit 103 may set the reached illuminance matching the upper limit. When the second user, who wakes up later, is a female, the control pattern adjusting unit 103 may set the reached illuminance matching the lower limit. The attributes including the age of the first and second users are stored in the memory 53 in advance.

The control pattern adjusting unit 103 may acquire a position of the second user. Based on the acquired position, the control pattern adjusting unit 103 may set the reached illuminance within the reached illuminance setting range between the determined upper limit and the lower limit. A camera installed in the bedroom captures an image of the second user asleep in bed. The server 5 recognizes a position of the second user from the image captured by the camera. When the second user is in a position of facing the lighting device 6, the control pattern adjusting unit 103 may sets a reached illuminance closer to the lower limit. When the second user is in a position of not facing the lighting device 6, the control pattern adjusting unit 103 may sets a reached illuminance closer to the upper limit.

The server 5 may determine whether the second user is in a position of lying on his or her back, based on an image captured by the camera. When the second user is in the position of lying on his or her back, the control pattern adjusting unit 103 may sets a reached illuminance closer to the lower limit. When the second user is not in the position of lying on his or her back, the control pattern adjusting unit 103 may sets a reached illuminance closer to the upper limit.

According to the first embodiment, the control pattern adjusting unit 103 adjusts the reached illuminance at the scheduled wake-up time of the first user. The method of control pattern adjustment according to the present disclosure, however, is not limited to this. When the sleep state of the second user is deeper than the reference sleep state, the control pattern adjusting unit 103 may adjust an acquired stimulus control pattern so that a point of time of intensifying a stimulus, the point of time being indicated by the acquired stimulus control pattern, arrives earlier.

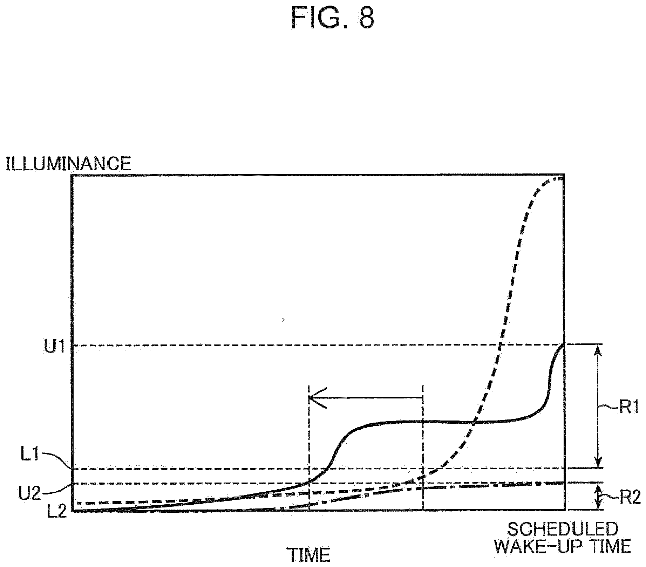

FIG. 8 depicts another example of the stimulus control patterns for controlling the stimulus given to the first user who wakes up first, according to first embodiment of the present disclosure. In FIG. 8, the vertical axis represents illuminance while the horizontal axis represents time. In FIG. 8, a broken line represents the ordinary reference stimulus control pattern in the case where one person is asleep in bed. According to the reference stimulus control pattern, light control is carried out in such a way as to gradually increase illuminance from a point of time a given time (e.g., 30 minutes) prior to a scheduled wake-up time and to sharply raise the illuminance to its maximum in a period between a point of time a given time (e.g., 3 minutes) prior to the scheduled wake-up time, that is, right before the scheduled wake-up time and the scheduled wake-up time. Through this control, an optical stimulus given to the first user facilitates the first user's waking up with a refreshed feeling.

In FIG. 8, a continuous line represents a stimulus control pattern that is applied to a case where the sleep state of the second user is the deep sleep state, and a single-dot chain line represents a stimulus control pattern that is applied to a case where the sleep state of the second user is the light sleep state. A range between the upper limit U1 and the lower limit L1 of the reached illuminance is a reached illuminance setting range R1, while a range between the upper limit U2 and the lower limit L2 of the reached illuminance is a reached illuminance setting range R2.

As shown in FIG. 8, when the sleep state of the second user is the deep sleep state, the control pattern adjusting unit 103 may adjust a stimulus control pattern so that a point of time of raising the illuminance sharply, the point of time being indicated by the stimulus control pattern, arrives earlier. The control pattern adjusting unit 103 may adjust the stimulus control pattern in such a way as to raise the illuminance to a given illuminance darker than the reached illuminance at a first point of time given time prior to the scheduled wake-up time, to maintain the given illuminance for a given period, and to sharply raise the illuminance to the reached illuminance at a second point of time a given time prior to the scheduled wake-up time, that is, right before the scheduled wake-up time.

In this manner, the stimulus control pattern is adjusted so that the point of time of raising the illuminance sharply, the point of time being indicated by the stimulus control pattern, arrives earlier. This allows the sleep state of the first user to be changed early to the light sleep state. Even if the reached illuminance is darker than the ordinary illuminance level, therefore, the reached illuminance stimulates the first user to wake up at the scheduled wake-up time.

Even when the sleep state of the second user is the light sleep state, the control pattern adjusting unit 103 may adjust the stimulus control pattern so that the point of time of raising the illuminance sharply, the point of time being indicated by the stimulus control pattern, arrives earlier.

The upper limit and the lower limit of the reached illuminance according to the first embodiment may be given values obtained from knowledge of lighting control carried out at the time of the user's waking up. The reached illuminance may be updated, as the equipment control system is used, in accordance with a degree of the first user's awakening from sleep or a degree of a body motion the second user makes at the time of the first user's waking up. For example, the sleep state determining unit 102 may determine whether the first user has awoken from sleep at the scheduled wake-up time. When the first user has awoken from sleep at the scheduled wake-up time, the control pattern adjusting unit 103 maintains the current reached illuminance. When the first user has failed to awake from sleep at the scheduled wake-up time, on the other hand, the control pattern adjusting unit 103 may change the current reached illuminance in such a way as to bring it closer to the upper limit.

For example, when the degree of the body motion the second user makes at the scheduled wake-up time of the first user is equal to or larger than a given value, the control pattern adjusting unit 103 may change the current reached illuminance in such a way as to bring it closer to the lower limit. When the degree of the body motion the second user makes at the scheduled wake-up time of the first user is smaller than the given value, the control pattern adjusting unit 103 may change the current reached illuminance in such a way as to bring it closer to the upper limit.

According to the first embodiment, the control pattern adjusting unit 103 adjusts the reached illuminance at the scheduled wake-up time of the first user in accordance with the sleep state of the second user. The method of illuminance adjustment according to the present disclosure, however, is not limited to this. The current illuminance may be changed in accordance with the sleep state of the second user.

According to the first embodiment, the control pattern acquiring unit 101 may acquire a stimulus control pattern, based on the scheduled wake-up time of the second user who wakes up later. The equipment control unit 104 may generates a control command for controlling the operation of the lighting device 6 according to the stimulus control pattern for the second user acquired by the control pattern acquiring unit 101. In such a case, after the passage of the scheduled wake-up time of the first user, who wakes up first, the lighting device 6 operates according to the stimulus control pattern for the second user, who wake up later, until the scheduled wake-up time of the second user arrives.

When one lighting device is present, it is necessary that after the passage of the scheduled wake-up time of the first user, who wakes up first, the operation of the lighting device be controlled based on the scheduled wake-up time of the second user, who wakes up later. However, when the first lighting device and the second lighting device that separately light up the first user and the second user, respectively, are present, the operation of the second lighting device can be controlled based on the scheduled wake-up time of the second user, who wakes up later, regardless of whether the scheduled wake-up time of the first user, who wakes up first, has passed or not.

### (Second Embodiment)

According to the first embodiment, the control pattern adjusting unit 103 sets the reached illuminance within the reached illuminance setting range between the determined upper limit and the lower limit, based on the difference between the scheduled wake-up time of the first user and the scheduled wake-up time of the second user. According to a second embodiment, the control pattern adjusting unit sets the reached illuminance within the reached illuminance setting range between the determined upper limit and the lower limit, based on priority level information indicating which of the first user and the second user is to be given priority.

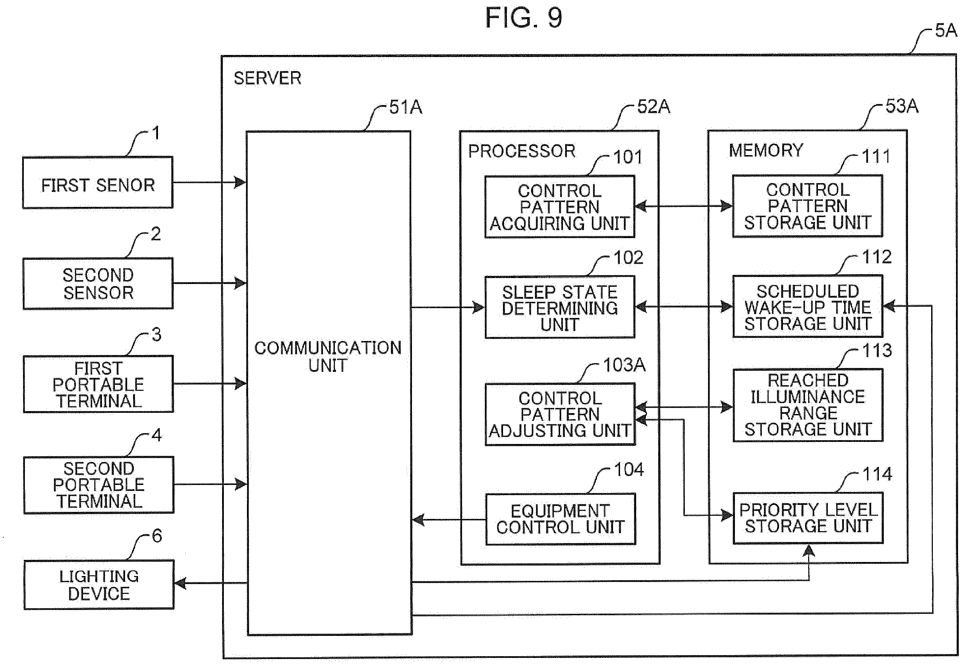

FIG. 9 depicts a configuration of a server according to the second embodiment of the present disclosure.

A server 5A includes a communication unit 51A, a processor 52A, and a memory 53A. The processor 52A has the control pattern acquiring unit 101, the sleep state determining unit 102, a control pattern adjusting unit 103A, and the equipment control unit 104. The memory 53A has the control pattern storage unit 111, the scheduled wake-up time storage unit 112, the reached illuminance range storage unit 113, and a priority level storage unit 114. In the second embodiment, the same constituent elements as described in the first embodiment are denoted by the same reference marks and are not described further.

The first portable terminal 3 receives input of a scheduled wake-up time of the first user and receives also input of priority level information indicating which of the first user and the second user is to be given priority. The first portable terminal 3 sends the scheduled wake-up time of the first user and the priority level information, to the server 5.

The second portable terminal 4 may receive input of priority level information indicating which of the first user and the second user is to be given priority. When both the first portable terminal 3 and the second portable terminal 4 send different versions of the priority level information to the server 5, the server 5 may adopt the latest version of the priority level information.

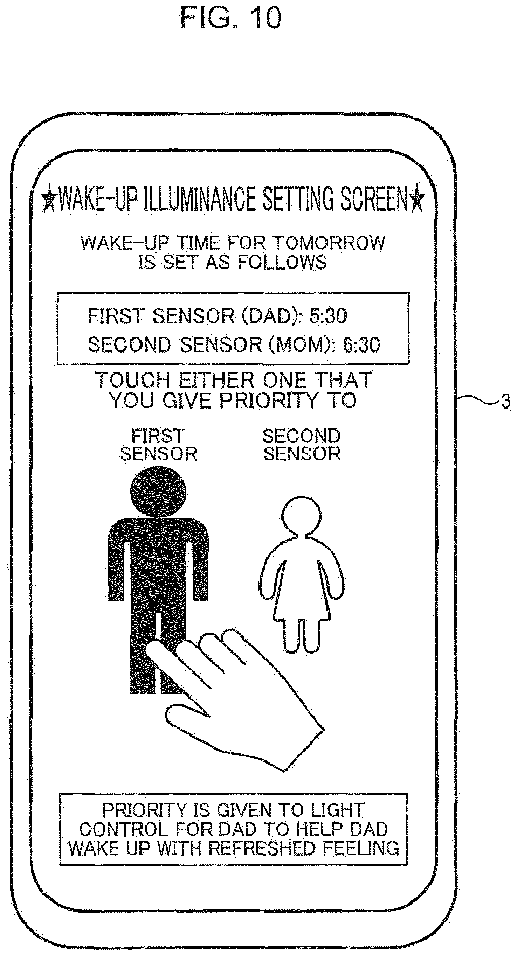

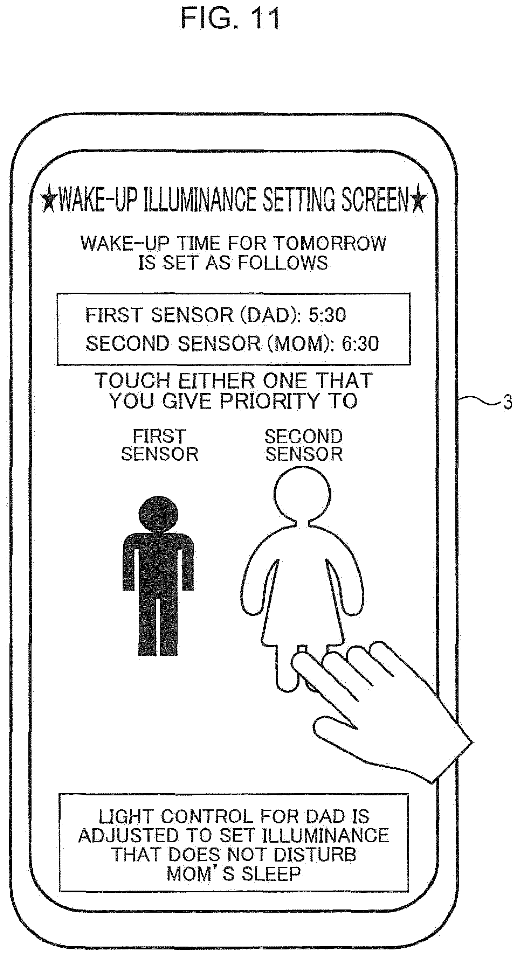

FIG. 10 depicts an example of a priority level input screen that is displayed on the first portable terminal according to the second embodiment of the present disclosure when the first user is given priority, and FIG. 11 depicts an example of a priority level input screen that is displayed on the first portable terminal according to the second embodiment of the present disclosure when the second user is given priority.

In the second embodiment, an operation carried out in a case where the first user sets a priority level to follow in controlling lighting to a person who wakes up first, using application software installed in the first portable terminal 3, will be described. In FIGS. 10 and 11, "dad" refers to the first user and "mom" refers to the second user.

On the priority level input screen shown in FIG. 10, the first user, who wakes up first, gives priority to the first user over the second user, in order to ensure that the first user will wake up pleasantly on the next day. On the priority level input screen shown in FIG. 11, in contrast, the first user, who wakes up first, gives priority to the second user over the first user, in order to ensure that the second user will wake up pleasantly on the next day. The priority level input screen receives input of priority level information indicating which of the first user and the second user is to be given priority. The first user chooses an image of the user to be given priority from images of the first user and the second user displayed on the priority level input screen and touches the chosen image. This action determines which of the first user and the second user is to be given priority.

The first portable terminal 3 transmits priority level information indicating which of the first user and the second user is to be given priority, the priority level information being input by the first user, to the server 5.

The communication unit 51A of the server 5A receives the scheduled wake-up time of the first user and the priority level information that are sent by the first portable terminal 3.

The priority level storage unit 114 stores the priority level information indicating which of the first user and the second user is to be given priority, the priority level information being input by the first portable terminal 3. The priority level storage unit 114 stores the priority level information such that it is associated with identification information for identifying the lighting device 6. The communication unit 51A stores the received priority level information in the priority level storage unit 114.

According to the second embodiment, because the second user wakes up later, the control pattern adjusting unit 103A acquires a sleep state of the second user, who wakes up later, from the sleep state determining unit 102. Based on the sleep state of the second user, the control pattern adjusting unit 103A adjusts a stimulus control pattern acquired by the control pattern acquiring unit 101. The control pattern adjusting unit 103A refers to the reached illuminance range storage unit 113, and determines a reached illuminance setting range in accordance with the sleep state of the second user determined by the sleep state determining unit 102.

The control pattern adjusting unit 103A acquires the priority level information indicating which of the first user and the second user is to be given priority, from the priority level storage unit 114. Based on the acquired priority level information, the control pattern adjusting unit 103A adjusts the reached illuminance (output) of the stimulus control pattern within the reached illuminance setting range (output setting range). For example, when the priority level information indicates that the first user is to be given priority, the control pattern adjusting unit 103A sets the reached illuminance matching the upper limit of the reached illuminance setting range. When the priority level information indicates that the second user is to be given priority, in contrast, the control pattern adjusting unit 103A sets the reached illuminance matching the lower limit of the reached illuminance setting range. The reached illuminance to be set, if it is within the reached illuminance setting range, may be an illuminance value different from the upper limit or lower limit. When the priority level information is not used, for example, the reached illuminance matching the median of the reached illuminance setting range may be set.

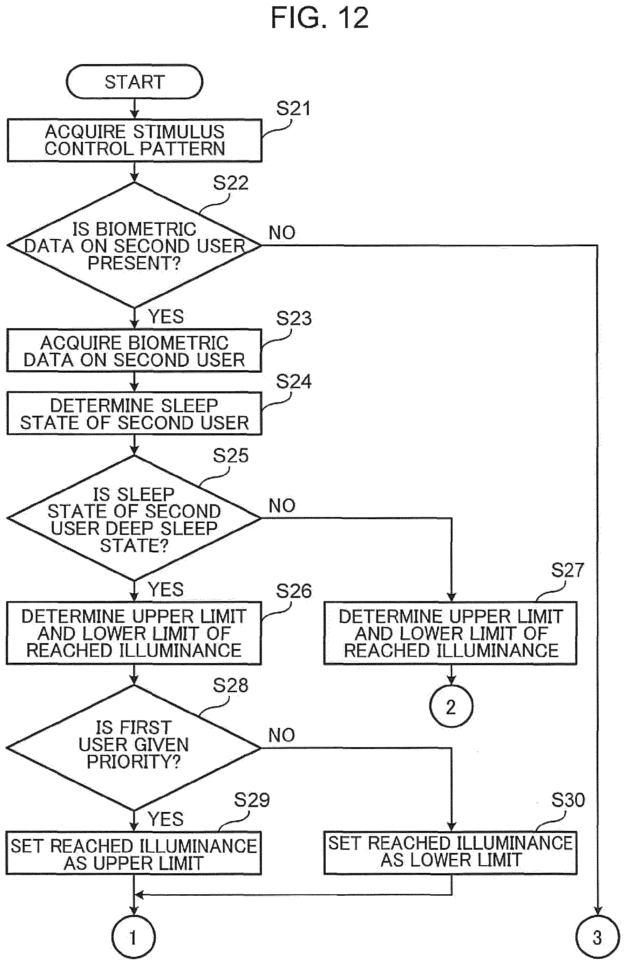

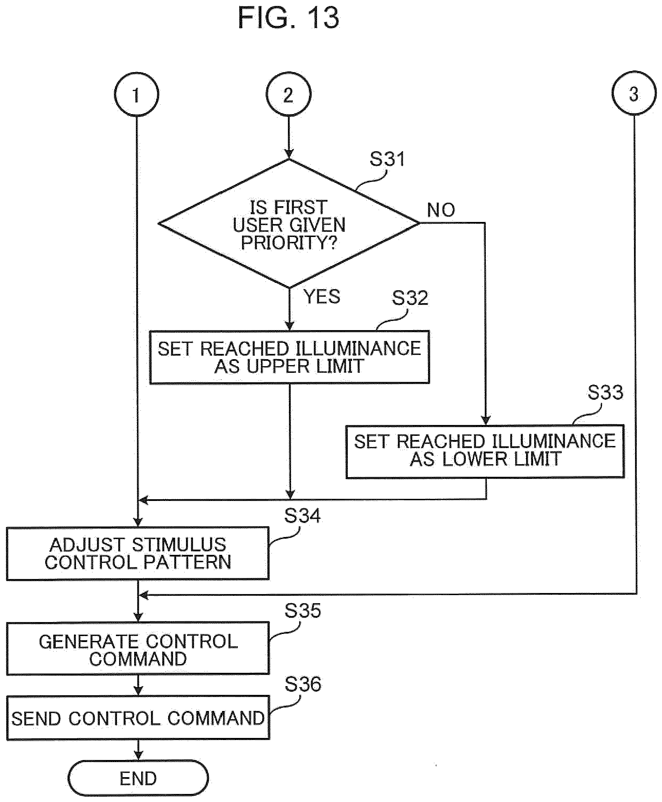

FIG. 12 is a first flowchart for explaining an operation of the server according to the second embodiment of the present disclosure, and FIG. 13 is a second flowchart for explaining an operation of the server according to the second embodiment of the present disclosure.

Processes of steps S21 to S27 are the same as the processes of steps S1 to S7 shown in FIG. 7, and therefore will not be described further.

After the upper limit and the lower limit of the reached illuminance that are associated with the deep sleep state are determined at step S26, the control pattern adjusting unit 103A refers to the priority level information stored in the priority level storage unit 114, and determines whether the first user is given priority (step S28). When determining that the first user is given priority (YES at step S28), the control pattern adjusting unit 103A sets the reached illuminance of a stimulus control pattern for the first user, as the upper limit of the reached illuminance setting range (step S29). When the table shown in FIG. 4 is used, the control pattern adjusting unit 103A sets the reached illuminance, as the upper limit specified in the table, i.e., 500 lux.

When determining that the first user is not given priority, that is, determining that the second user is given priority (NO at step S28), in contrast, the control pattern adjusting unit 103A sets the reached illuminance of the stimulus control pattern for the first user, as the lower limit of the reached illuminance setting range (step S30). When the table shown in FIG. 4 is used, therefore, the control pattern adjusting unit 103A sets the reached illuminance, as the lower limit specified in the table, i.e., 30 lux.

After the upper limit and the lower limit of the reached illuminance that are associated with the light sleep state are determined at step S27, the control pattern adjusting unit 103A refers to the priority level information stored in the priority level storage unit 114, and determines whether the first user is given priority (step S31). When determining that the first user is given priority (YES at step S31), the control pattern adjusting unit 103A sets the reached illuminance of a stimulus control pattern for the first user, as the upper limit of the reached illuminance setting range (step S32). When the table shown in FIG. 4 is used, the control pattern adjusting unit 103A sets the reached illuminance, as the upper limit specified in the table, i.e., 10 lux.

When determining that the first user is not given priority, that is, determining that the second user is given priority (NO at step S31), in contrast, the control pattern adjusting unit 103A sets the reached illuminance of the stimulus control pattern for the first user, as the lower limit of the reached illuminance setting range (step S33). When the table shown in FIG. 4 is used, therefore, the control pattern adjusting unit 103A sets the reached illuminance, as the lower limit specified in the table, i.e., 0 lux.

Processes of steps S34 to S36 are the same as the processes of steps S9 to S11 shown in FIG. 7, and therefore will not be described further.

According to the first and second embodiments, at a point of arrival of the scheduled wake-up time of the first user, the first portable terminal 3 may receive input of a preferred illuminance that the first user wishes to be exposed when waking up.

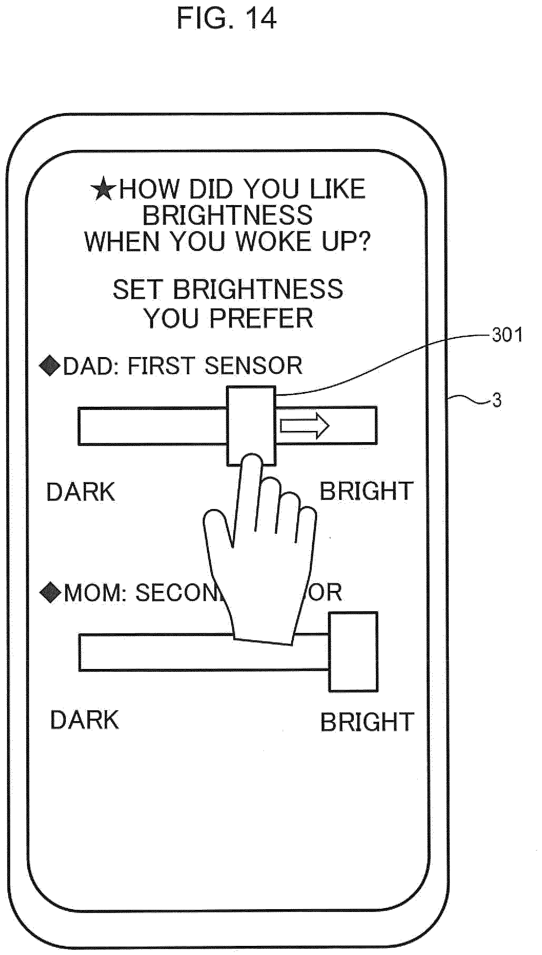

FIG. 14 depicts an example of an illuminance input screen that receives input of a preferred illuminance that the first user wishes to be exposed when waking up, the illuminance input screen being displayed on the first portable terminal according to the first and second embodiments of the present disclosure. In FIG. 14, "dad" refers to the first user and "mom" refers to the second user.

When the scheduled wake-up time of the first user has arrived, the first portable terminal 3 displays the illuminance input screen. On the illuminance input screen shown in FIG. 14, a tab image 301 is displayed, the tab image 301 indicating a reached illuminance at the scheduled wake-up time of the first user, the reached illuminance being within a reached illuminance setting range associated with a sleep state of the second user. The first user slides the tab image 301 in accordance with a preferred illuminance of the lighting device 6 that the first user wishes to be exposed when waking up, thereby adjusts the reached illuminance to the preferred illuminance between the lower limit and the upper limit of the reached illuminance setting range.

In this case, the communication unit 51 sends the lower limit and the upper limit of the reached illuminance of a control pattern adjusted by the control pattern adjusting unit 103 and the reached illuminance, to the first portable terminal 3. When the scheduled wake-up time of the first user has arrived, the first portable terminal 3 displays an illuminance input screen generated by using the incoming upper limit and the lower limit of the reached illuminance and the reached illuminance. When the first user has changed the reached illuminance, the first portable terminal 3 sends the changed reached illuminance to the server 5. The communication unit 51 stores the incoming changed reached illuminance, together with the sleep state of the second user and a given condition based on which the original reached illuminance is set, in the reached illuminance range storage unit 113. The changed reached illuminance is equivalent to the preferred illuminance for the first user.

The control pattern adjusting unit 103 may acquire at least one of the preferred illuminance for the first user and the preferred illuminance for the second user. Based on the acquired preferred illuminance, the control pattern adjusting unit 103 may adjust the reached illuminance (output) of the stimulus control pattern within the reached illuminance setting range (output setting range).

In this manner, the user is allowed to input the preferred illuminance the user wishes to be exposed when waking up and to make minor adjustment of the reached illuminance in the range between the upper limit and the lower limit, in accordance with the preferred illuminance.

According to the first and second embodiments of the present disclosure, the lighting device 6 controls a stimulus for helping the first user in waking up. Stimulus control according to the present disclosure, however, is not limited to this. The lighting device 6 may control a stimulus for helping the first user in falling asleep.

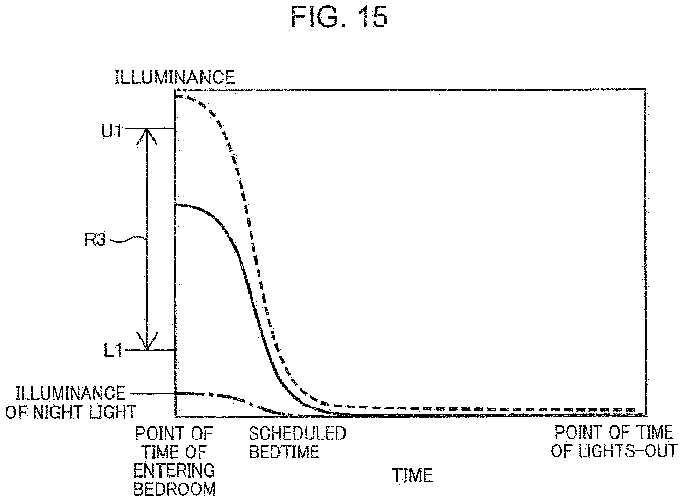

FIG. 15 depicts an example of stimulus control patterns for controlling a stimulus given to the first user who falls asleep later, according to a modification of the first and second embodiments. In FIG. 15, the vertical axis represents illuminance and the horizontal axis represents time. In FIG. 15, a broken line represents the ordinary reference stimulus control pattern in the case where one person is asleep in bed. According to the reference stimulus control pattern, illuminance control is carried out in such a way as to gradually decrease illuminance from its maximum in a period between a point of time of entering the bedroom and a scheduled bedtime, to sharply reduce the illuminance in a period between the scheduled bedtime and a first time a given time (e.g., 10 minutes) after the scheduled bedtime, to gradually decrease the illuminance in a period between the first time and a second time given time (e.g., 20 minutes) after the first time, and to turn the lighting device off at the second time. This control induces the user to comfortably fall asleep through adjusted optical stimulus.

The scheduled bedtime is input to the first portable terminal 3 by the first user. A camera for identifying a user who enter the bedroom is set in a location outside the bedroom and close to a bedroom door. The server analyzes an image captured by the camera, thereby identifies an order in which the first and second users go to bed and identifies a time at which the first user, who goes to bed later, enters the bedroom.

The control pattern adjusting unit 103 refers to a table stored in an initial illuminance range storage unit, determines an initial illuminance setting range for an initial illuminance of a stimulus control pattern, according to a sleep state of the second user, and determines an initial illuminance within the determined initial illuminance setting range.

The initial illuminance range storage unit stores, for each type of the lighting device, a table in which a sleep state of the second user, who goes to bed first, is associated with an initial illuminance setting range indicating the upper limit U1 and the lower limit L1 of an initial illuminance, which should be given to the first user, who goes to bed later, at a point of time of the first user's entering the bedroom. According to this modification, only one lighting device 6 is disposed in the main bedroom.

The upper limit U1 indicating an illuminance (e.g., 800 lux) darker than the ordinary illuminance and the lower limit L1 indicating an illuminance (e.g., 30 lux) brighter than the illuminance of a night light are associated with the deep sleep state. The ordinary illuminance is equivalent to the maximum illuminance of the lighting device 6, which is, for example, 1000 lux. The illuminance of a night light is, for example, 10 lux. The upper limit U1 represents an illuminance level that is, for example, 80% of the ordinary illuminance.

An illuminance (e.g., 10 lux) equal to the illuminance of a night light is associated with the light sleep state.

The upper limit and the lower limit of the initial illuminance may be given values that are obtained from knowledge of lighting control carried out at the time of user's falling asleep. The initial illuminance may be updated in accordance with the first user's preference.

In FIG. 15, a continuous line represents a stimulus control pattern that is applied to a case where the sleep state of the second user is the deep sleep state, and a single-dot chain line represents a stimulus control pattern that is applied to a case where the sleep state of the second user is the light sleep state. A range between the upper limit U1 and the lower limit L1 of the initial illuminance is an initial illuminance setting range R3.

As shown in FIG. 15, the initial illuminance of the stimulus control pattern for the case where the sleep state of the second user is the deep sleep state is smaller than the initial illuminance of the reference stimulus control pattern. The initial illuminance of the stimulus control pattern for the case where the sleep state of the second user is the light sleep state is smaller than the initial illuminance of the stimulus control pattern for the case where the sleep state of the second user is the deep sleep state.

Thus, when the first user and the second user sleep in the same space, it is possible to induce the first user, who goes to bed later, to comfortably fall asleep without disturbing the second user's sleep, the second user going to bed first.

The control pattern adjusting unit 103 may adjust the stimulus control pattern, based on an environmental condition of the bedroom in which the first and second users sleep. For example, the morning sunlight coming into the bedroom causes a change in the illuminance in the bedroom. For this reason, the control pattern adjusting unit 103 may adjust the stimulus control pattern, based on the illuminance in the bedroom in which the first and second users sleep. The equipment control system may further include an illuminance sensor that is disposed in the bedroom to measure the illuminance. When the scheduled wake-up time is behind the time of sunrise, the control pattern adjusting unit 103 may adjust the stimulus control pattern in such a way as to delay a point of time of sharply raising the illuminance and quickly raise the illuminance in a short period. The lighting device 6 may continuously acquire a current illuminance measured by the illuminance sensor. When a current illuminance measured by the illuminance sensor is higher than a current illuminance indicated by the stimulus control pattern, the lighting device 6 may not perform its lighting operation. When the current illuminance measured by the illuminance sensor is equal to or lower than the current illuminance indicated by the stimulus control pattern, in contrast, the lighting device 6 may perform its lighting operation.

In the first and second embodiments of the present disclosure, control of the lighting device 6 that lights up a space is described. The present disclosure, however, is not limited to control of the lighting device 6 but may be applied also to control of a sound output device that outputs sounds into a space, an air freshener diffuser that diffuses an air freshener in a space, and an airflow output device that sends airflows into a space.

For example, in controlling the sound output device, the control pattern adjusting unit 103 sets a reached sound volume at a scheduled wake-up time for a case where the sleep state of the second user is the deep sleep state, as a sound volume that is smaller than a normal sound volume and is larger than a reached sound volume at a scheduled wake-up time for a case where the sleep state of the second user is the light sleep state. In controlling the air freshener diffuser, the control pattern adjusting unit 103 sets a reached fragrance at the scheduled wake-up time for the case where the sleep state of the second user is the deep sleep state, as a fragrance that is weaker than a normal fragrance and is stronger than a reached fragrance at the scheduled wake-up time for the case where the sleep state of the second user is the light sleep state. In controlling the airflow output device, the control pattern adjusting unit 103 sets a reached airflow volume at the scheduled wake-up time for the case where the sleep state of the second user is the deep sleep state, as an airflow volume that is smaller than a normal airflow volume and is larger than a reached airflow volume at the scheduled wake-up time for the case where the sleep state of the second user is the light sleep state. In the cases of these devices different from the lighting device, the output of the stimulus control pattern at the scheduled wake-up time may be adjusted in accordance with the difference between the scheduled wake-up time of the first user and the scheduled wake-up time of the second user, the body motion of the second user, or a preferred one input by the first user or second user.

Through this adjustment, when the first user and the second user sleep in the same space, it is possible to induce the first user to comfortably fall asleep or wake up without disturbing the second user's sleep. The second user is allowed to sleep comfortably until the scheduled wake-up time arrives, without being disturbed by the first user who falls asleep later or wakes up first. Thus, the second user is also prompted to comfortably fall asleep or wake up as the first user is.

In the above embodiments, each constituent element is provided as dedicated hardware. However, each constituent element may be provided as software by executing a software program for creating each constituent element. Each constituent element may be created by a program executing unit, such as a CPU or processor, which reads a software program out of a recording medium, such as a hard disc or semiconductor memory, and executes the program.

Some or all of functions of the device according to the embodiments of the present disclosure are realized typically in the form of large-scale integrated (LSI) circuits. These LSI circuits may each be constructed as a separate single chip or may be integrated into a single chip carrying some or all of the LSI circuits. Integrated circuits realizing the functions of the device are not limited to LSI circuits but may be provided as dedicated circuits or general-purpose processors. In circuit integration, a field programmable gate array (FPGA), which can be programmed after manufacturing of an LSI circuit, or a reconfigurable processor, which allows reconfiguration of connections/settings of circuit cells inside an LSI circuit, may be used.

Some or all of functions of the device according to the embodiments of the present disclosure may be realized by a processor, such as a CPU, that executes programs.

All the numerical values mentioned above are exemplary numerical values used for explaining the present disclosure. The exemplary numerical values, therefore, do not put any limitations on the present disclosure.

The order of execution of the steps making up the above flowcharts is an example of the order indicated for specifically explaining the present disclosure. A different order of execution of the steps, therefore, may be adopted in a range in which the different order of execution offers the same effect as the order of execution shown in the flowcharts offers. Some of the above steps may be executed simultaneously (in parallel) with other steps.

The information processing method, the information processing program, and the information processing system according to the present disclosure induce the first user to comfortably fall asleep or wake up without disturbing the second user's sleep when the first user and the second user sleep in the same space, and are therefore useful as the information processing method, the information processing program, and the information processing system that control the operation of one or more devices that control a stimulus for helping the first user and the second user in falling asleep or waking up.

This application is based on Japanese Patent application No. 2019-011972 filed in Japan Patent Office on January 28, 2019, the contents of which are hereby incorporated by reference.

Although the present invention has been fully described by way of example with reference to the accompanying drawings, it is to be understood that various changes and modifications will be apparent to those skilled in the art. Therefore, unless otherwise such changes and modifications depart from the scope of the present invention hereinafter defined, they should be construed as being included therein.

## Claims

1. An information processing method comprising, by a computer:
acquiring a stimulus control pattern for controlling a stimulus that one or more devices give to a first user to induce the first user to fall asleep or wake up;
acquiring a sleep state of a second user who is in a same space in which the first user is;
adjusting the acquired stimulus control pattern, based on the sleep state of the second user; and
controlling an operation of the one or more devices, according to the adjusted stimulus control pattern.

2. The information processing method according to claim 1, wherein in adjustment of the stimulus control pattern, the acquired stimulus control pattern is adjusted so that a stimulus given to the first user when the sleep state is lighter than a reference sleep state becomes weaker than a stimulus given to the first user when the sleep state is deeper than the reference sleep state.

3. The information processing method according to claim 2, wherein in adjustment of the stimulus control pattern, the acquired stimulus control pattern is adjusted so that the stimulus becomes weaker than a stimulus determined by the acquired stimulus control pattern.

4. The information processing method according to any one of claims 1 to 3, wherein in adjustment of the stimulus control pattern, the acquired stimulus control pattern is adjusted so that a point of time of intensifying the stimulus in the acquired stimulus control pattern arrives earlier when the sleep state is deeper than the reference sleep state.

5. The information processing method according to any one of claims 1 to 4, wherein in adjustment of the stimulus control pattern, an output setting range for the acquired stimulus control pattern is adjusted.

6. The information processing method according to claim 5, further comprising acquiring a first scheduled wake-up time of the first user and a second scheduled wake-up time of the second user, wherein
in adjustment of the stimulus control pattern, an output of the acquired stimulus control pattern is adjusted within the output setting range, based on a difference between the first scheduled wake-up time and the second scheduled wake-up time.

7. The information processing method according to claim 5, further comprising acquiring an attribute or preference of at least one of the first user and the second user, wherein
in adjustment of the stimulus control pattern, an output of the acquired stimulus control pattern is adjusted within the output setting range, based on the acquired attribute or preference.

8. The information processing method according to claim 5, further comprising acquiring priority level information indicating which of the first user and the second user is to be given priority, wherein
in adjustment of the stimulus control pattern, an output of the acquired stimulus control pattern is adjusted within the output setting range, based on the acquired priority level information.

9. The information processing method according to claim 5, further comprising acquiring a position of the second user, wherein
in adjustment of the stimulus control pattern, an output of the acquired stimulus control pattern is adjusted within the output setting range, based on the acquired position.

10. The information processing method according to any one of claims 1 to 9, wherein
the one or more devices include a first device associated with the first user and a second device associated with the second user, and
in control of the operation, an operation of the first device is controlled according to the adjusted stimulus control pattern.

11. The information processing method according to any one of claims 1 to 10, wherein the one or more devices include at least one of a lighting device that lights up the space, a sound output device that outputs a sound into the space, an air freshener diffuser that diffuses an air freshener in the space, and an airflow output device that sends airflow into the space.

12. An information processing program for causing a computer to:
acquire a stimulus control pattern for controlling a stimulus that one or more devices give to a first user to induce the first user to fall asleep or wake up;
acquire a sleep state of a second user who is in a same space in which the first user is;
adjust the acquired stimulus control pattern, based on the sleep state of the second user; and
control an operation of the one or more devices, according to the adjusted stimulus control pattern.

13. An information processing system comprising:
a stimulus control pattern acquiring unit that acquires a stimulus control pattern for controlling a stimulus that one or more devices give to a first user to induce the first user to fall asleep or wake up;
a sleep state acquiring unit that acquires a sleep state of a second user who is in a same space in which the first user is;
an adjusting unit that adjusts the acquired stimulus control pattern, based on the sleep state of the second user; and
a control unit that controls an operation of the one or more devices, according to the adjusted stimulus control pattern.
